# EUROPEAN PATENT APPLICATION

(11) **EP 4 834 202 A1**
(43) Date of publication of application: **04.11.2026**
(21) Application number: 25173443.0
(22) Date of filing: 30.04.2025
(51) Int. Cl.: A61B 5/00

(54) **DATA SIGNAL FOR BIOMEDICAL SIGNALS**

(71) Applicant: Koninklijke Philips N.V., 5656 AE Eindhoven (NL)
(72) Inventor: TSONEVA, Tsvetomira Kirova, 5656 AG Eindhoven (NL); JELFS, Sam Martin, 5656 AG Eindhoven (NL); OOMEN, Arnoldus Werner Johannes, 5656 AG Eindhoven (NL)
(74) Representative: Philips Intellectual Property & Standards

(57) **Abstract**

An apparatus generates a biomedical data signal representing a plurality of biomedical signal comprising biomedical signals. A cluster circuit (109) generates a set of clusters of biomedical signals by applying a clustering process to the plurality of biomedical signals where the clustering process clustered biomedical signals depending on signal property similarities between the plurality of biomedical signals. A data encoder (103) generates encoded compressed data by encoding the plurality of biomedical signals. The data encoder (103) applies relative encoding to biomedical signals within a first cluster of the set of clusters. A data signal generator (105) generates the biomedical data signal to comprise the compressed encoded data and an indication of the set of clusters. A decoder may use to the indication of the set of clusters to perform the reverse operation to generate the biomedical signals.

## Description

### FIELD OF THE INVENTION

The invention relates to a data signal for biomedical signals and specifically, but not exclusively, to generating and/or processing such a signal.

### BACKGROUND OF THE INVENTION

The capturing, storing, distributing, and processing of biomedical data has become increasingly important in recent decades as recording such signals has progressed from being performed almost exclusively for short term measurements in dedicated clinical environments, such as hospitals, to becoming increasingly prevalent in the personal space with biomedical measurements often being made continuously over very long periods. For example, the cardiology field has evolved from mainly considering short heart rate waveforms collected within the clinic to using chest-worn outpatient devices that are routinely worn for multiple days, and which collect continuous data for long durations. Such measurements typically include simultaneous measurements at different positions on the body and result in a multichannel biomedical signal with a high level of information content.

Consequently, the requirements for data storage, distribution, and processing have grown exponentially. Accordingly, there is a desire to effectively represent captured biomedical signals to facilitate and reduce the requirements for storing, distributing, and/or processing the signals. There is further a desire for such approaches to be suitable for efficient recreation of the biomedical signals from such data. However, whereas various proprietary approaches have been proposed for encoding or compressing (and for correspondingly decoding or decompressing) biomedical waveform signals used by various manufacturers, current approaches tend to be suboptimal and tend to not provide ideal performance. Further, current approaches tend to be complex and/or be targeted on specific biomedical waveform signals resulting in specific requirements and limited application.

Hence, an improved approach for representing multichannel biomedical signals would be advantageous. In particular, an approach allowing increased flexibility, improved performance, increased quality, reduced complexity, reduced data rate, increased accuracy/reduced distortion of encoding/decoding/compressing/decompressing operations, reduced storage requirements, reduced bandwidth/data rate for biomedical waveform signals, reduced computational load, facilitated implementation, and/or improved performance would be advantageous.

### SUMMARY OF THE INVENTION

Accordingly, the invention seeks to preferably mitigate, alleviate or eliminate one or more of the above-mentioned disadvantages singly or in any combination.

According to an aspect of the invention, there is provided an apparatus for generating a biomedical data signal, the apparatus comprising: a receiver arranged to receive a plurality of biomedical signals; a cluster circuit arranged to generate a set of clusters of biomedical signals by applying a clustering process to the plurality of biomedical signals, the clustering process being arranged to cluster biomedical signals of the plurality of biomedical signals depending on signal property similarities between the plurality of biomedical signals; a data encoder arranged to generate encoded compressed data by encoding the plurality of biomedical signals, the data encoder being arranged to apply relative encoding to biomedical signals within a first cluster of the set of clusters; and a data signal generator arranged to generate the biomedical data signal to comprise the compressed encoded data and an indication of the set of clusters.

The invention may allow advantageous generation of a biomedical data signal representing a plurality of biomedical signals. It may in many embodiments allow an improved data rate to quality trade-off, and may in many embodiments provide advantageous compression/encoding of the biomedical signals. The invention may allow a reduced bandwidth/data rate for compressed encoded data representing a multichannel biomedical signal. In many embodiments, the storage requirements and/or required communication bandwidth may be reduced substantially. The approach may allow efficient and practical implementation and/or may reduce complexity and processing/computational requirements.

The biomedical waveform signal may specifically be an electrocardiography (ECG), electroencephalography (EEG), or electromyography (EMG) signal but any other bioelectrical signals, such as, but not limited to, Electrocorticogram (ECoG), Electrooculogram (EOG), Electroretinogram (ERG), Fetal electrocardiography (fECG), Electrohysterogram (EHG), Electrodermal Activity (EDA), photoplethysmogram (PPG) or Electrogastrogram (EGG) could also apply.

Generating the encoded compressed data may include applying a data compression algorithm to the biomedical signals. The data encoder may be arranged to apply a data compression algorithm to the biomedical signals.

In some embodiments, a plurality, and possibly all, biomedical signals represent a measured differential voltage between a common reference electrode and a measurement electrode for the biomedical signal. In some embodiments, a first biomedical signal represents a measured differential voltage between a first pair of electrodes, and a second biomedical signal represents a measured differential voltage between a second pair of electrodes, the first pair of electrodes not including any electrode of the second pair of electrodes.

The signal property similarities may be determined in accordance with any similarity measure, such as a similarity measure indicative of a (cross)correlation between biomedical signals of the plurality of biomedical signals. The signal property similarities may be signal correlation measures (between biomedical signals of the plurality of biomedical signals).

The data encoder may be arranged to encode at least a first biomedical signal belonging to the first cluster of the set of clusters relative to a second biomedical signal belonging to the first cluster.

The data encoder may be arranged to encode at least a first biomedical signal belonging to the first cluster of the set of clusters as a difference signal indicative of a difference between the first biomedical signal and a signal predicted from a second biomedical signal belonging to the first cluster.

The data encoder may be arranged to apply a predictive encoding of at least a first biomedical signal predicted from a second biomedical signal, the first biomedical signal and the second biomedical signal being selected as belonging to a same cluster.

In many embodiments, the clustering is dependent on statistical properties for the biomedical signals.

According to an optional feature of the invention, the cluster circuit is arranged to repeatedly perform the clustering process to update the set of clusters.

This may provide particularly advantageous operation and/or performance in many embodiments and scenarios. The cluster circuit may be arranged to repeat the clustering process at periodic intervals, or e.g., intermittently/non-periodically, e.g., in response to events occurring or being detected.

According to an optional feature of the invention, the data encoder is arranged to generate encoded reference signal data for the first reference signal; and the data signal generator is arranged to generate the biomedical data signal to comprise the encoded reference signal data.

This may provide particularly advantageous operation and/or performance in many embodiments and scenarios.

According to an optional feature of the invention, the apparatus comprises a transform circuit arranged to apply a signal transform to the plurality of biomedical signals prior to the clustering process.

This may provide particularly advantageous operation and/or performance in many embodiments and scenarios. It may typically provide reduced complexity and/or a reduced data rate.

In many embodiments, the transform circuit is arranged to apply the signal transform to the plurality of biomedical signals prior to the generation of the encoded compressed data.

According to an optional feature of the invention, the apparatus is arranged to apply a sequence of operations to the plurality of biomedical signals to generate the encoded compressed data; and wherein the data signal generator is arranged to include an indication of a position of the clustering process in the sequence.

This may provide particularly advantageous operation and/or performance in many embodiments and scenarios.

The sequence may specifically include applying a signal transform and the clustering could be performed before or after this. The sequence may specifically include applying a quantization operation and the clustering could be performed before or after this. The sequence may specifically include the encoding of the data encoder, and the clustering is typically performed before this prediction/relative encoding. The sequence may specifically include an entropy encoding and the clustering may be performed before this entropy encoding.

According to an optional feature of the invention, the plurality of biomedical signals comprises biomedical signals of at least two different types.

This may provide particularly advantageous operation and/or performance in many embodiments and scenarios.

The types may include two or more of: Electrooculogram (EOG) signals, Electroretinogram (ERG) signals, Electrodermal activity (EDA) signals, Blood Pressure (BP) signals, Respiration Rate (RR) signals, Pulse Oximetry (SpO2) signals, Galvanic Skin Response (GSR) signals, Photoplethysmogram (PPG) signals, Body Temperature signals, Electrogastrogram (EGG) signals, Phonocardiogram (PCG) signals, Ballistocardiogram (BCG) signals, Dermal Activity signals, Actigraphy signals, Functional Near-Infrared Spectroscopy (fNIRS) signals, Nasal Airflow signals, Electrohysterography (EHG) signals, and Tocodynamometry (TOCO) signals.

According to an optional feature of the invention, the clustering process is arranged to cluster the biomedical signals of the plurality of biomedical signals depending on measurement positions for the plurality of biomedical signals.

This may provide particularly advantageous operation and/or performance in many embodiments and scenarios. The measurement positions may be positions on (typically the body of) a test subject/patient.

According to an optional feature of the invention, the data encoder is arranged to individually, for at least two clusters of the set of clusters, select an encoding process from a plurality of encoding processes in dependence on signal properties of biomedical signals comprised in the at least two clusters.

This may provide particularly advantageous operation and/or performance in many embodiments and scenarios.

In some embodiments, the data encoder is arranged to generate encoded compressed data by applying different encoding processes to biomedical signals of at least two clusters of the set of clusters.

According to an optional feature of the invention, the data encoder is arranged to time align biomedical signals of at least a first cluster of the set of clusters.

This may provide particularly advantageous operation and/or performance in many embodiments and scenarios.

According to an optional feature of the invention, the cluster circuit is arranged to select the clustering process from a set of candidate clustering processes, and the data signal generator is arranged to generate the biomedical data signal to comprise an indication of the clustering process.

This may provide particularly advantageous operation and/or performance in many embodiments and scenarios.

According to an optional feature of the invention, the apparatus further comprises: a determination circuit arranged to derive a first reference signal from biomedical signals of a first cluster of the set of clusters; a compensation circuit arranged to generate compensated biomedical signals by compensating at least some of the biomedical signals of a first cluster for the first reference signal; and wherein the data encoder is arranged to generate encoded compressed data for the first cluster by compressing the at least some of the biomedical signals of the first cluster.

This may provide particularly advantageous operation and/or performance in many embodiments and scenarios.

According to another aspect of the invention, there is provided an apparatus for generating a plurality of biomedical signals, the apparatus comprising: a receiver arranged to receive a biomedical data signal comprising compressed encoded data for a plurality of biomedical signals and a cluster indication indicative of a clustering of the plurality of biomedical signals into a set of clusters, each cluster comprising biomedical signals clustered in dependence on signal property similarities between the plurality of biomedical signals, the encoded data comprising relative encoding data for at least some biomedical signals within a same cluster; and a decoder arranged to generate the plurality of biomedical signals by decoding the encoded data in dependence on the cluster indication.

The relative encoding data for at least a first biomedical signal of the same cluster may be encoding data representing a difference signal indicative of a difference between the first biomedical signal and a signal generated by prediction from a second biomedical signal, the second biomedical signal being a biomedical signal of the same cluster.

According to another aspect of the invention, there is provided a method of generating a plurality of biomedical signals, the method comprising: receiving a biomedical data signal comprising compressed encoded data for a plurality of biomedical signals and a cluster indication indicative of a clustering of the plurality of biomedical signals into a set of clusters, each cluster comprising biomedical signals clustered in dependence on signal property similarities between the plurality of biomedical signals, the encoded data comprising relative encoding data for at least some biomedical signals within a same cluster; and generating the plurality of biomedical signals by decoding the encoded data in dependence on the cluster indication.

According to another aspect of the invention, there is provided a method of generating a biomedical data signal, the method comprising: receiving a plurality of biomedical signals; generating a set of clusters of biomedical signals by applying a clustering process to the plurality of biomedical signals, the clustering process being arranged to cluster biomedical signals of the plurality of biomedical signals depending on signal property similarities between the plurality of biomedical signals; generating encoded compressed data by encoding the plurality of biomedical signals, the data encoder being arranged to apply relative encoding to biomedical signals within a first cluster of the set of clusters; and generating the biomedical data signal to comprise the compressed encoded data and an indication of the set of clusters.

According to another aspect of the invention, there is provided a biomedical data signal for representing a plurality of biomedical signals, the biomedical data signal comprising: compressed encoded data for a plurality of biomedical signals; and a cluster indication indicative of a clustering of the plurality of biomedical signals into a set of clusters, each cluster comprising biomedical signals clustered in dependence on signal property similarities between the plurality of biomedical signals; and wherein the encoded data comprise relative encoding data for at least some biomedical signals within a same cluster.

These and other aspects, features and advantages of the invention will be apparent from and elucidated with reference to the embodiment(s) described hereinafter.

### BRIEF DESCRIPTION OF THE DRAWINGS

Embodiments of the invention will be described, by way of example only, with reference to the drawings, in which
FIG. 1 illustrates some elements of an example of an apparatus for generating a biomedical data signal in accordance with some embodiments of the invention;
FIG. 2 illustrates some elements of an example of an apparatus for generating biomedical signals from a biomedical data signal in accordance with some embodiments of the invention;
FIG. 3 illustrates some elements of an example of a clustering approach for an apparatus in accordance with some embodiments of the invention;
FIG. 4 illustrates some elements of an example of a clustering approach applied to a plurality of biomedical signals;
FIG. 5 illustrates some elements of examples of apparatuses in accordance with some embodiments of the invention;
FIG. 6 illustrates some elements of an example of an encoder circuit for an apparatus for generating a biomedical data signal in accordance with some embodiments of the invention; and
FIG. 7 illustrates some elements of a possible arrangement of a processor for implementing elements of an apparatus of FIG. 1 or 2 in accordance with some embodiments of the invention.

### DETAILED DESCRIPTION OF SOME EMBODIMENTS OF THE INVENTION

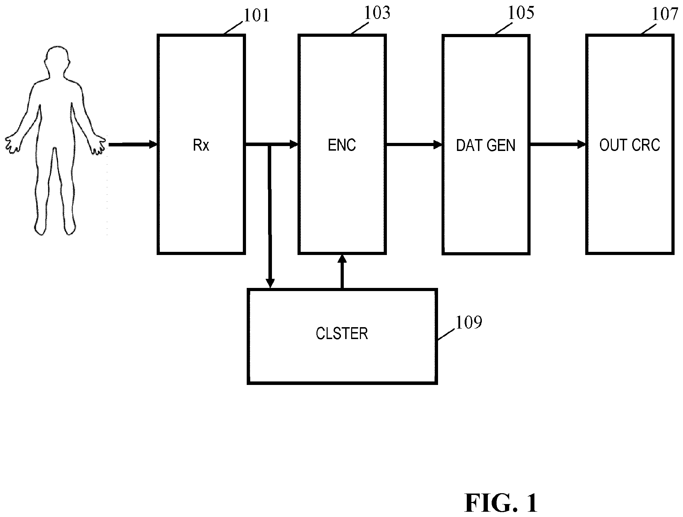

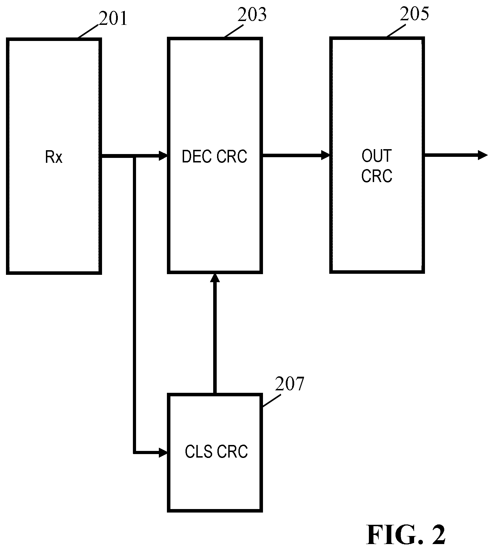

FIG. 1 illustrates an example of an apparatus for generating a biomedical data signal comprising data representing a plurality of biomedical signals, such as signals of one or more multichannel biomedical signals. FIG. 2 illustrates an example of an apparatus for generating/recreating a plurality of biomedical signals (such as signals of one or more multichannel biomedical signals) from a biomedical data signal representing such biomedical signals. The apparatus of FIG. 1 will be referred to as the encoder apparatus and the apparatus of FIG. 2, which specifically may receive the biomedical data signal from the encoder apparatus, will be referred to as the decoder apparatus.

The encoder apparatus comprises a receiver 101 which receives a plurality of biomedical signals. For example, the receiver 101 may receive one or more multichannel biomedical signals. In some such embodiments, each of the biomedical signals may represent a measured differential voltage between two measurement electrodes. The measurement electrodes may be positioned in contact with/attached to a patient's/subject's body and each biomedical signal may be generated by measuring the voltage difference between two attached electrodes. The number of electrodes and/or channels may depend on the specific application and usage and may range from a few electrodes/channels up to hundreds of electrodes/channels. In some embodiments, each biomedical signal may be a differential voltage relative to a common electrode. Thus, the biomedical signals may all represent a differential voltage with respect to the same reference electrode. In other embodiments, each biomedical signal may be a differential voltage between separate pairs of electrodes and thus a different reference electrode may be used for each biomedical signal. In other embodiments, the biomedical signals may be divided into groups with the signals of each group being a differential voltage relative to a common reference or electrode for the group, but with this common reference or electrode being different for different groups. For example, reference electrodes may be distributed across the measurement area, and the differential voltage for electrodes may be measured relative to the reference electrode closest to the given electrode, or a linear combination of all electrodes surrounding the electrode/group, etc.

In other embodiments, the biomedical signals may be other types of signals determined by other measurements, such as for example measurements that are generated from optical measurements.

In many embodiments, the biomedical signals may include an electrocardiography (ECG) (multichannel) signal which specifically may be provided from a sensor arrangement comprising a plurality of electrodes that are positioned on the subject's body to pick up electrical signals relating to the heart operation.

In some embodiments, the biomedical signals may include an electroencephalography (EEG) (multichannel) signal which specifically may be provided from a sensor comprising a number of electrodes that are positioned on the subject's head to pick up electrical signals relating to brain activity.

In some embodiments, the biomedical signals may be an electromyography (EMG) (multichannel) signal which specifically may be provided from a sensor comprising a plurality of electrodes that are positioned on the subject's body to pick up electrical signals relating to muscle activity of the person.

In some embodiments, the biomedical signals may include a magnetoencephalography (MEG) signal which specifically may be provided from magnetometers that are positioned around the subject's head to detect magnetic fields associated with neural activity.

In some embodiments, the biomedical signals can include electrooculography (EOG) data, collected from electrodes placed near the eyes to monitor eye movements through detection of electrical potential changes caused by eye activity.

In some embodiments, the biomedical signals may also include photoplethysmography (PPG) signals, utilizing optical sensors typically attached to the skin to measure volumetric changes in blood circulation, offering insights into heart rate and vascular health. Optionally, the PPG signals may be obtained via camera or other vision systems.

In some embodiments, the biomedical signals may also include respiration data which can be acquired through sensors positioned to detect changes in airflow, chest expansion, or abdominal movement, providing valuable information about the subject's respiratory dynamics.

In some embodiments, the biomedical signals may include accelerometer data, which can be captured using sensors capable of detecting acceleration forces along multiple axes in order to measure bodily movement and orientation.

In some embodiments, the biomedical signals can include Galvanic skin response (GSR) data gathered from electrodes applied to the skin to record variations in electrical conductance, reflecting changes in sweat gland activity.

It will be appreciated that the multichannel biomedical signal may in different embodiments be any suitable bioelectrical signals. Examples of other possible biomedical signals include Electroretinogram (ERG) signals, Electrodermal activity (EDA) signals, Blood Pressure (BP) signals, Respiration Rate (RR) signals, Pulse Oximetry (SpO2) signals, Body Temperature signals, Electrogastrogram (EGG) signals, Phonocardiogram (PCG) signals, Ballistocardiogram (BCG) signals, Dermal Activity signals, Actigraphy signals, Functional Near-Infrared Spectroscopy (fNIRS) signals, Nasal Airflow signals, Electrohysterography (EHG) signals, and Tocodynamometry (TOCO) signals.

Each of the biomedical signals may specifically be a time domain signal with a time domain waveform that reflects the changes in the measured value, such as in the differential voltage from an electrode relative to a reference electrode.

The encoder apparatus proceeds to process the biomedical signals to generate a biomedical data signal which includes data that represents/describes the biomedical signals. The biomedical data signal may be generated to comprise encoded data representing the biomedical signals (which itself may be represented/received as data values, such as e.g., represented by digital signals representing measured voltages after an A/D conversion).

The encoder apparatus comprises a data encoder 103 which is arranged to generate encoded compressed data by encoding the plurality of biomedical signals. The data encoder 103 may be a lossy encoding and may include a data compression to reduce the number of bits required to represent the biomedical signals.

The data encoder 103 may be arranged to perform a compression process to generate compressed data from the biomedical signals. The compression may be an encoding/compression which results in a representation of the biomedical signals which has a reduced data rate/size relative to the biomedical signals themselves. The compression may in some cases be a lossy compression and may in some cases be a lossless compression.

The data encoder 103 may first perform a compression operation to generate compressed data which may then be encoded as part of being included in the biomedical data signal. Thus, the data of the biomedical data signal may be encoded compressed data.

The encoding/compression may include operations to remove redundancy by applying lossless coding techniques such as entropy coding, etc. In many cases, the data encoder 103 may also be arranged to remove irrelevancy from the compensated biomedical signals by applying lossy coding techniques such as quantization, noise substitution, etc.

Redundancy removal may preserve the perfect reconstruction of the multichannel biomedical signal. After irrelevancy removal, perfect reconstruction is generally no longer feasible.

The removal of irrelevance is often obtained e.g., by simple thresholding (quantization, e.g., setting coefficients less than a given threshold to zero). For entropy coding various techniques and methods are available, such as Huffman coding or Arithmetic coding.

As will be described in more detail later, the data encoder 103 may specifically be arranged to perform relative or predictive encoding where one or more of the biomedical signals is encoded relative to another of the biomedical signals. Specifically, a first signal may be predicted from a second signal and the data encoder 103 may generate a difference signal representing the difference between the first signal and the predicted signal. It may then proceed to encode the difference signal rather than the first signal to generate encoded compressed data representing the first signal.

The data encoder 103 is coupled to a data signal generator 105 which generates the biomedical data signal to comprise the compressed encoded data. The data signal generator 105 may generate a bitstream in accordance with a suitable data format that includes data fields which can carry the compressed encoded data. The data signal generator 105 may generate the biomedical data signal to include metadata. It may further include suitable overhead data required or desired e.g., for communication, synchronization etc.

In some embodiments, the data signal generator 105 may be coupled to an optional output circuit 109 which may be arranged to output the biomedical data signal to any suitable destination and in any suitable way. In many embodiments, the output circuit may comprise functionality for storing the biomedical data signal in a suitable memory/store (which e.g., may be a portable store) and/or for communicating the biomedical data signal to a remote destination over a suitable communication channel, such as e.g., via a network such as the Internet. In particular, the output circuit 107 may be arranged to transmit the biomedical data signal to the decoder apparatus of FIG. 2. The biomedical data signal may be distributed or communicated in any suitable way including real time communication, storage on storage devices, communication over a network etc.

The encoder apparatus further comprises a cluster processor or circuit 109 which is arranged to perform a clustering process that generates a set of clusters from the biomedical signals. The cluster circuit 109 is arranged to perform the clustering in dependence on signal property similarities for the biomedical signals. The clustering process may group biomedical signals into clusters of biomedical signals that have suitably similar signal properties. The clustering process may be based on a similarity measure or metric which reflects a suitable property that is desired for the resulting clusters. A suitable similarity measure or metric may for example be based on a cross correlation between signals. For example, in a first iteration, a cross-correlation between all pairs of biomedical signals may be generated and a first set of clusters may be generated comprising pairs of biomedical signals with the lowest cross-correlation (e.g., subject to this being below a threshold). Further iterations may then be performed to further combine the clusters of previous iterations based on the cross-correlations between selected signals of the clusters. The selected cluster signal may for example be determined as the biomedical signal of the cluster having the smallest combined correlation with all other biomedical signals of the cluster. The process may be iterated until a set of clusters has been determined that meet a given criterion, such as that each cluster has a maximum cross-correlation between any pair of biomedical signals that is below a threshold and with any further clustering resulting in the threshold being exceeded.

The cluster circuit 109 may thus cluster the biomedical signals depending on signal property similarities between the plurality of biomedical signals and may generate a set of clusters with each cluster comprising biomedical signals having similar signal properties (in accordance with any suitable similarity measure, such as specifically a correlation measure). The clusters may be generated to comprise biomedical signals that have low cross-correlation (specifically a cross correlation below a threshold).

The cluster circuit 109 is coupled to the data encoder 103 which is provided with the information of the result of the clustering process, and specifically the data encoder 103 receives data indicating the resulting clusters and the biomedical signal comprised in each cluster.

The data encoder 103 proceeds to perform the encoding based on the clustering. Specifically, the relative encoding of the biomedical signal is based on the clustering with the encoding of one or more biomedical signal in a cluster being a relative encoding relative to one or more other biomedical signal within the same cluster. In many embodiments, the data encoder 103 may specifically be arranged to not use any biomedical signal not belonging to the same cluster as a first biomedical signal for the relative encoding of the first biomedical signal. The relative encoding may thus in many embodiments be restricted to not comprise, consider, or include biomedical signals of different clusters.

For example, in some embodiments, the data encoder 103 may for each cluster proceed to determine a reference signal from the biomedical signal of the cluster. The reference signal may typically be one of the biomedical signals of the cluster, but could in some embodiments be generated by combining biomedical signals of the cluster in accordance with a suitable algorithm. In many embodiments, the biomedical signal with the lowest/highest accumulated/combined correlation with all other biomedical signals of the cluster may be selected as a reference signal. This reference signal may then be encoded (including being compressed) by the data encoder 103 without any reference to any other biomedical signal, i.e., it may be a non-relative/ non-predictive encoding. Each of the other biomedical signals of the cluster may however be encoded relative to the reference biomedical signal. For a given biomedical signal of the cluster, a predicted signal may be generated from the reference biomedical signal and the data encoder 103 may encode (and compress) the resulting difference signal resulting from removing the predicted signal from the biomedical signal.

The predictive encoding data for at least a first biomedical signal of the same cluster may be encoding data representing a difference signal indicative of a difference between the first biomedical signal and a signal generated by prediction from a second biomedical signal, the second biomedical signal being a biomedical signal of the same cluster.

The data encoder may thus be arranged to encode at least a first biomedical signal belonging to the first cluster of the set of clusters relative to a second biomedical signal belonging to the first cluster. The data encoder may be arranged to encode at least a first biomedical signal belonging to the first cluster of the set of clusters as a difference signal indicative of a difference between the first biomedical signal and a signal predicted from a second biomedical signal belonging to the first cluster. The data encoder may be arranged to apply a predictive encoding of at least a first biomedical signal predicted from a second biomedical signal, the first biomedical signal and the second biomedical signal being selected as belonging to a same cluster.

The cluster circuit 109 is further coupled to the data signal generator 105 which is also fed the data on the clustering process. In particular, it is fed the data providing information of the clusters, including which biomedical data signals are included in the different clusters. The data signal generator 105 is arranged to include an indication of the result of the cluster process in the biomedical data signal. Specifically, the data signal generator 105 may include metadata in the biomedical data signal, which indicates the determined clusters and which biomedical signals belong to each cluster. For example, the metadata may for each cluster indicate a biomedical signal that is used as a reference signal and all the biomedical signals that have been encoded relative to this signal.

The decoder apparatus of FIG. 2 comprises a decoder receiver 201 which receives a biomedical data signal from any internal or external data source. For example, the decoder receiver 201 may extract the biomedical data signal from an internal storage or from a portable storage medium, may receive it via a network interface (e.g., via an Internet connection) etc. The decoder receiver 201 may be arranged to parse/interpret a received bitstream to extract the relevant data. The decoder receiver 201 may specifically receive the biomedical data signal from the encoder apparatus of FIG. 1 (or indeed a corresponding signal may be received from another source).

The decoder apparatus further comprises a decoding circuit 203 arranged to generate the biomedical signals by decoding the compressed encoded data of the biomedical data signal. The decoding circuit 203 receives the encoded compressed data from the decoder receiver 201 as well as the indication of the clustering results. It then proceeds to decode the encoded compressed data based on this indication. In particular, it performs the reverse operation of the encoding process by the data encoder 103, and specifically it may proceed to perform a relative/predictive decoding of the biomedical signals that are indicated by the clustering indication to be encoded relative to another biomedical signal of the same cluster.

As an example, the indication may for a given cluster indicate that one of the biomedical signals is a reference signal that is not relatively encoded, and this may be decoded based on the encoded compressed data without reference to any other biomedical signal. It may then proceed to decode the remaining biomedical signal of the same cluster as a relative decoding based on the reference signal. For example, using the same algorithm as used by the data encoder 103, a biomedical signal may be predicted from the received and decoded reference signal, and this may be combined with a difference signal to result in the decoded biomedical signal, where the difference signal is generated by decoding the corresponding encoded compression data.

The decoding circuit 203 may thus recreate the biomedical signals from the biomedical data signal. In the specific example, the biomedical signals may be fed to an output circuit 205 which may proceed to process, present, store, or in any way use or manipulate the generated biomedical signals. For example, it may store the biomedical signals in a suitable memory.

In many embodiments, the decoder apparatus may further comprise a decoder cluster circuit 207 which is arranged to determine the clustering of biomedical signals as performed by the encoder apparatus. In some embodiments, the decoder cluster circuit 207 may simply determine the clustering from the direct metadata provided in the biomedical data signal and directly indicating which biomedical signals belong to the same cluster and are relatively encoded.

For example, in some embodiments, the meta data may contain a flag indicating there are clusters used, for example 0 means no clusters are used, and 1 means clusters are used. This flag may be followed by a field that indicates the number of clusters used. This number could be coded in a fixed number of bits. For example, 3 bits would allow a maximum of 8 clusters being used ('000' representing 1 and '111' representing 8). Alternatively the flag indicating whether there are clusters used could be combined into this 3 bit field. In that case, '000' could represent no clusters are used. Value '001' could represent 1 cluster used up to '111' indicating 7 clusters are used. The fixed number of bits could be replaced or combined with alternative coding methods such as Huffman, which exploit the probability of occurrences of a certain number of clusters used. Specific (Huffman) codes could be reserved to code outliers for the number of clusters used, which could be relevant in the case a very large number of biomedical channels is encoded.

In the decoder apparatus, the declustering process may loop over the number of clusters and for each cluster receive an indication of the channel index so that the correct order can be reproduced again. These channel indices may be encoded differentially with respect to each other or in other well-known ways in order to reduce the overhead for the clustering metadata.

The approach may provide a highly advantageous coding (and typically compression) of a plurality of biomedical signals. An improved quality/accuracy to data rate trade-off can be achieved in many situations and for many scenarios and applications. Further, the approach can be implemented with low complexity and/or computational resource requirements.

The approach may exploit that the inherent redundancy in many biomedical signals (e.g., with similar information is captured across multiple channels due to the common source, volume conduction and external factors) can be advantageous used in the context of data compression. The described approach may advantageously exploit this redundancy to reduce the amount of data that needs to be stored or transmitted without (significant) loss of signal quality.

In some embodiments, the approach may employ a method for clustering biomedical signals (such as specifically bioelectrical signals) such that clusters are formed based on the similarity and redundancy of the signals. By grouping channels that exhibit high correlation or another (statistical) measure of similarity, the clustering approach may enhance the efficiency of data compression. The method may consider statistical features to determine similarity which may make it applicable to a range of different signals allowing the encoder apparatus and decoder apparatus to handle various types of biomedical signals, including combinations thereof. This flexibility allows the method to be applied to very different types of biomedical signals, such as EEG, ECG, and EMG, among others. It may advantageously in many embodiments allow for a seamless integration of multiple types of signals within a unified framework. In many embodiments and scenarios, the approach not only improves compression ratios, but also enhances computational performance and resource utilization, providing significant benefits for the processing and analysis of multichannel biomedical signals.

The approach may provide a method designed to enhance the compression ratio for both lossless and lossy compression of multichannel biomedical signals. The approach may involve determining/designing/selecting a similarity metric used to measure similarity/correlation between different biomedical signals. Based on this metric, a similarity/distance matrix may be constructed to represent the relationships between the biomedical signals. Subsequently, the number of clusters may be determined, either statically or dynamically, and a clustering algorithm may be applied to group the signals with similar characteristics. The clustered signals may subsequently be encoded. The clustering process can be integrated as either a preprocessing step or applied at a later stage in the data processing pipeline. Different clusters could also be passed down through different branches of the data processing pipeline.

The encoder may typically be arranged to dynamically update the clustering and the data encoder 103 may be arranged to dynamically adapt the encoding to reflect changes in the clusters. Thus, the cluster circuit 109 may be arranged to repeatedly perform the clustering process to update the set of clusters. Such updates may for example be periodic or event driven. For example, updated clustering may be performed at regular intervals such as e.g., every 1-100 seconds. For example, the biomedical signals may be divided into time segments with a given (e.g., fixed) duration and for each segment, a clustering may be performed (based on the biomedical signals in that segment) and the signals of that segment may be encoded based on that clustering. As another example, in some embodiments, the biomedical signals may be evaluated to detect if there is a suitable change in the signal properties (e.g., a simple measure such as a signal level may be considered or variability over a predefined period of time). If the change is detected, the cluster circuit 109 may be triggered to perform a new clustering to update the clusters.

It will be appreciated that a number of different clustering algorithms and/or similarity measures are known and that any suitable approach may be used. Indeed, in many embodiments, the cluster circuit 109 may be arranged to switch between different clustering algorithms and/or similarity measures. For example, in some embodiments, the biomedical signals may be evaluated at regular intervals and a cluster algorithms and/or similarity measure may be selected for the current signal properties.

The similarity measure for a clustering process may be a measure of how the distance or likeness between data points (in this case the biomedical signals) is quantified, which is fundamental for forming meaningful clusters. Various distance or similarity measures could be appropriate for the biomedical signals and different clustering methods. A suitable similarity measure helps to ensure that clusters are coherent and meaningful, indicating points within the same cluster being genuinely similar and those in different clusters being distinct.

Common similarity measures include:
Correlation Coefficient: Measures linear relationship between two data points.
Euclidean Distance: Measures the straight-line distance between two points in Euclidean space.
Mean-square error: Measures the average squared difference between points.
Manhattan Distance: Measures the sum of absolute differences between points.
Cosine Similarity: Measures the cosine of the angle between two vectors.
Mutual Information (MI): Measures how much knowing one channel reduces uncertainty about the other.
Dynamic Time Warping (DTW): Measures similarity between temporal sequences by aligning points and minimizing the temporal distortions.

Any of these similarity measures or their combination could be applied to the biomedical signals and used for the clustering process by the cluster circuit 109.

A large number of clustering algorithms are known that group data points based on their similarities, enabling the identification of natural structures within data. Most of these work by calculating the distance or similarity between data points using various metrics, such as the ones described above, and then partition the data into distinct groups or clusters such that points within the same cluster are more similar to each other than to those in other clusters. Examples of clustering algorithms suitable for biomedical signals include:
K-Means Clustering: Partitions the data into K clusters minimizing the intra-cluster variance assigning points to the nearest mean/centroid.
Hierarchical Clustering: builds a tree of clusters/dendrogram by progressively merging or splitting subsets of data.
Spectral Clustering: uses the eigenvalues of a similarity matrix to perform dimensionality reduction before applying clustering.
Density-Based Spatial Clustering of Applications with Noise (DBSCAN): clusters data based on density, identifying core points with many neighbors and expanding clusters from them. Does not require a predefined number of clusters.
Dynamic Time Warping (DTW) Clustering: measures similarity between temporal sequences by aligning them and minimizing the distance between points.
Gaussian Mixture Models: uses probabilities for clustering assuming that the data is generated from a mixture of several Gaussian
Wavelet Transform-Based Clustering: clustering based on frequency-domain characteristics.
Deep Learning-Based Clustering: Techniques such as Autoencoders and deep neural networks can learn representations of bio-signals and perform clustering based on those learned features.

It will be appreciated that other clustering methods are known and may be used in different embodiments. In some embodiments, the clustering process may be fixedly selected e.g., as one of the above. In some embodiments, the cluster circuit 109 may be arranged to dynamically switch between different clustering algorithms, e.g., depending on the signal properties. In many embodiments, the cluster circuit 109 may be selected depending on the type of the biomedical signals, such as e.g., different clustering algorithms may be selected depending on whether the biomedical signals include EEG, ECG, or EMG signals.

In some embodiments, the cluster circuit 109 may be arranged to combine a plurality of several clustering methods, e.g., a wavelet transform may be combined with hierarchical clustering.

The clustering may be used to identify and group biomedical signals with similar features in terms of shape, amplitude, functional relationships, temporal properties, etc. The encoding of the biomedical signals for one cluster may be a relative encoding that exploits such similarities to achieve and improved compression efficiency.

The number of clusters into which the biomedical signals are grouped may be different in different embodiments and scenarios, and may in some embodiments and scenarios be a fixed number, whereas it in other embodiments or scenarios may be dynamically varied. Determining the optimal number of clusters can be done statically or dynamically, for example depending on the number of channels or heuristic methods based on signal statistics.

Static approach: The number of clusters can be predetermined and set to a constant value in both the encoder and decoder. The exact number, suitable for any type of signal and not exceeding the number of channels, may e.g., be decided beforehand based on statistical analysis of bio signal data in general. Another possibility would be to set a specific number for each type of signal or depending on the total number of channels.

While the static approaches are computationally efficient, dynamic methods that adapt to the varying characteristics of the signals may often provide advantageous performance.

Dynamic approach: In some embodiments, statistical analysis may be used to determine the number of clusters needed/desired. Different approaches may leverage different statistical and computational techniques. Some non-limiting examples are listed below:
- Square root of channel number: Set the number of clusters to the square root of the number of channels
- Principal Component Analysis (PCA): Use PCA to determine how many principal components explain most of the variance, e.g., 90 % of variance, and select the number of clusters based on that.
- Elbow Method: Iterate over a number of possible clusters and compute a cost function, such as the sum of squared distances within clusters. Then plot the cost against the number of clusters and identify the optimal number of clusters at the point where the rate of decrease sharply goes down - resembling an "elbow" - below a predefined number.
- Silhouette Analysis: Measures the cohesion and separation of clusters, calculating silhouette scores to determine how tightly grouped and well-separated clusters are iterating over a number of possible clusters. The number of clusters with the highest average silhouette score is typically chosen.
- Gap Statistics: Compare the total within-cluster variation for different numbers of clusters with that expected under null reference distributions and select the number that maximizes the gap statistic.
- Bayesian Information Criterion (BIC) and Akaike Information Criterion (AIC): Evaluate the trade-off between model complexity and goodness-of-fit, with the number of clusters that minimizes BIC or AIC being preferred.
- Cross-Validation: Split the data into training and testing subsets, assess the stability and performance of clusters across multiple validations, identifying the number that consistently shows superior results.
- Hierarchical Clustering and Dendrogram Analysis: Visualize dendrograms to see different levels of cluster structures and select a cut-off point/level for the number of clusters.
- Mutual Information Reduction: Compute pairwise mutual information between channels and select a number of clusters where the average mutual information within clusters is below a predefined threshold.

The above list is not exhaustive and other methods for determining the optimal number of clusters may be used without detracting from the approach. Additionally, the combination of methods, or e.g., using multiple indexes and selecting the most common cluster number overall, is also a viable approach.

It should be noted that in some clustering algorithms, the number of clusters is not explicitly preselected but, instead, emerges naturally from the data during the clustering process.

As a specific example of a low complexity clustering approach, the cluster circuit 109 may proceed to create pairwise clusters so that biomedical signals are grouped in pairs of two. The method includes calculating a distance matrix based on a similarity measure, such as one of the indicated measures described above. Biomedical signals are then grouped by pairing those with the minimum distance. Once a pair is formed, the involved biomedical signals are removed from the distance matrix, and the process continues until all pairs are selected. Any residual biomedical signals that cannot be paired may be placed into a separate cluster. The size of the cluster may in this example be N = 2, but bigger groups could also be considered. The resulting number of groups/clusters is then equal to the number of channels divided by the size of group N.

In many embodiments, the cluster circuit 109 may be arranged to select the clustering process dependent on the biomedical signals. For example, the cluster circuit 109 may be arranged to select between different clustering processes for EEG, ECG, and EMG signals (etc.). Specifically, the cluster circuit 109 may be arranged to select the clustering process from a set of candidate clustering processes, for example dependent on the type of signal, dependent on signal properties (e.g., a degree of variation or similarity), the result of applying the different clustering processes etc. As an example, the list of candidate clustering processes may correspond to the list provided above (or a subset thereof). The clustering process may be selected as the one resulting in the most preferred result in accordance with any suitable measure.

The cluster circuit 109 may provide information to the data signal generator 105 indicating the clustering process that was used, and the data signal generator 105 may then generate the biomedical data signal to comprise an indication of the clustering process that was selected and used by the cluster circuit 109.

In such a case, the decoder may proceed to adapt the processing depending on the clustering process used. For example, different algorithms may tend to generate clusters with different properties which may result in different encoding approaches being most efficient. Depending on the clustering algorithm, the decoder may accordingly adapt the decoding process or a property thereof. For example, some certain clustering methods might lead to specific error types being more prevalent during compression and decompression and accordingly an error correcting code may be applied that is particularly suitable for the specific cluster algorithm. The decoder may then adapt the error correction applied at the decoder to match that suitable for the specific algorithm.

As another example, depending on the clustering process used, the metadata may with fewer bits signal to the decoder apparatus what the appropriate inverse process is.

In some embodiments, the cluster circuit 109 may be arranged to select the similarity measure dependent on the biomedical signals. For example, the cluster circuit 109 may be arranged to select between different similarity measures for EEG, ECG, and EMG signals (etc.). Specifically, the cluster circuit 109 may be arranged to select the similarity measure from a set of candidate similarity measures, for example dependent on the type of signal, dependent on signal properties (e.g., a degree of variation or similarity), the result of applying the different clustering processes etc. As an example, the list of similarity measures may correspond to the list provided previously (or a subset thereof). The similarity measure may be selected as the one resulting in the most preferred result in accordance with any suitable measure.

The cluster circuit 109 may provide information to the data signal generator 105 indicating the similarity measure that was used, and the data signal generator 105 may generate the biomedical data signal to comprise an indication of the similarity measure that selected and used by the cluster circuit 109.

In such a case, the decoder may proceed to adapt the processing dependent on the similarity measure. For example, the resulting clusters may have particular properties dependent on the similarity measure used, and this may affect the subsequent processing such as particularly the operation of the encoding process. For example, the relative encoding efficiency and error characteristics may depend on how the biomedical signals have been clustered, and on the specific similarities between biomedical signals of the same cluster, and thus may depend on the specific similarity measure. The decoder apparatus may accordingly be arranged to perform some adaptive processing dependent on knowledge of the similarity measure used. As a low complexity and specific example, if the similarity measure includes a requirement that the maximum level difference between two biomedical signals of the same cluster is below a given threshold, the decoder apparatus may verify that this requirement is indeed met for biomedical signals encoded relative to each other. If the decoded biomedical signals exhibit a level difference exceeding the threshold, it can be concluded that transmission errors (or other errors) have occurred. The decoder apparatus may then e.g., seek to compensate the signals, or may e.g., proceed to discard these as unreliable.

Some embodiments may include applying certain signal transformations to the time series biomedical signals before proceeding to clustering. Examples include:
- PCA, which reduces the number of dimensions in large datasets to principal components that retain most of the original variance.
- ICA, which decomposes a multivariate signal into independent non-Gaussian signals.
- Linear Discriminant Analysis, which projects the data to a lower-dimensional space, aiming to maximize separation between different classes.
- Wavelet Transform, which decomposes the signals into different time-frequency components.
- Fourier Transform, DCT, which convert time-domain signals to frequency-domain, highlighting periodic patterns.
- Log Transformation, which converts the data to logarithmic scale to compress wide-ranging data and reduce skewness.

Depending on specific signal properties within a cluster, different transformations or a combination of transformations could be selected.

By applying these transformations, or similar, the data can be better prepared for clustering, ensuring that important features are preserved, irrelevant variations are minimized, and the overall quality of the clusters is improved.

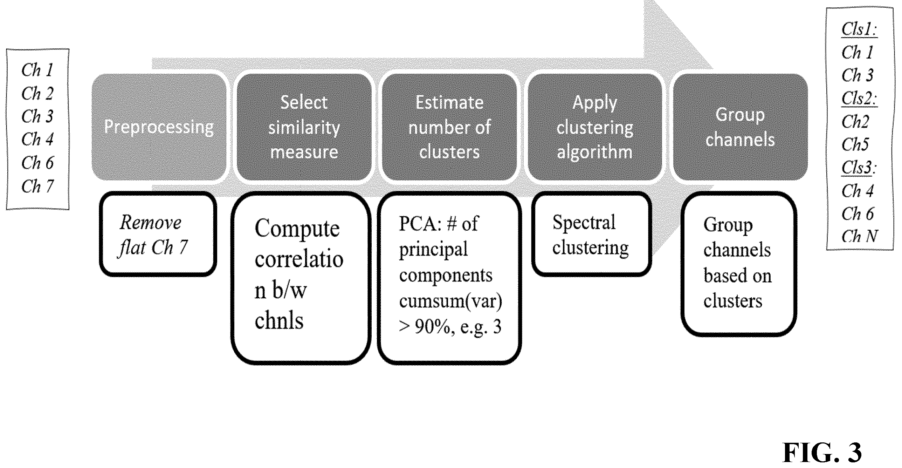

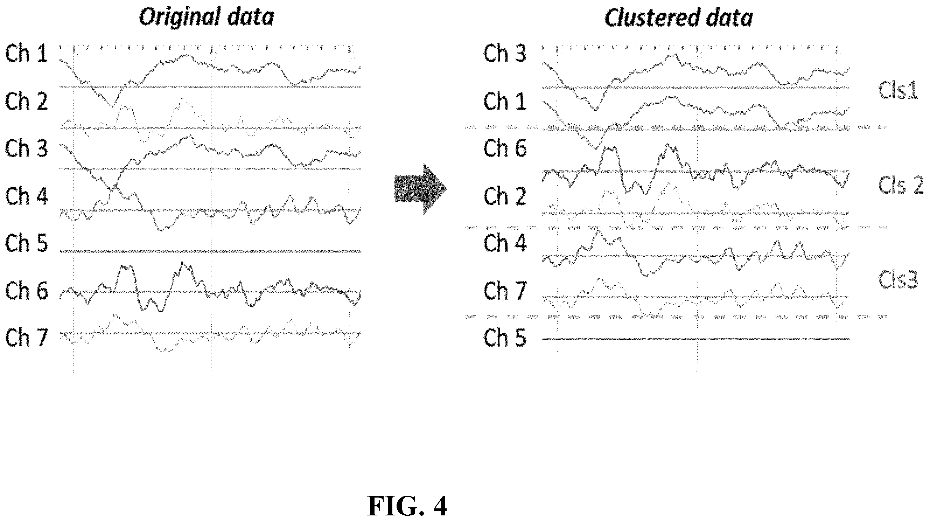

As indicated in FIG. 3, in many embodiments, a pre-processing may be applied to one or more of the biomedical signals prior to the clustering process. FIG. 4 illustrates some exemplary biomedical signals and how these may be clustered by the process of FIG.3. For example, during such an optional preprocessing step, the biomedical signals could be normalized or z-score standardized (see e.g., https://en.wikipedia.org/wiki/Feature_scaling), missing values or artifacts could be corrected and/or a certain transformation, e.g., Principal component analysis (PCA), Independent Component Analysis (ICA), wavelet, Direct cosine transform (DCT), etc., could be applied.

In particular, in many embodiments the receiver 101 may include a transform processor which is arranged to apply a signal transform to the biomedical signals prior to the clustering process and the generation of the encoded compressed data.

In many embodiments, the signal transform may be a time to frequency transform with the subsequent processing being performed in the frequency domain. For example, the signal transform may be a DCT (Discrete Cosine Transform), DST (Discrete Sine Transform), QMF (Quadrature Mirror Filter), FFT (Fast Fourier Transform), DWT (Discrete Wavelet Transform).

Such preprocessing may in many embodiments result in improved performance and, in particular, in a more efficient encoding process.

In some embodiments, the data signal generator 105 may further be arranged to include an indication of the signal transform that has been performed in the metadata of the biomedical data signal. In response to detecting this information, the decoder apparatus may be arranged to perform the reverse/complementary operation/transform, such as specifically a frequency to time transform.

In some embodiments, the decoder may be arranged to adapt other parts of the operation dependent on the indication. For example, if the decoder apparatus receives a biomedical data signal comprising an indication that a time to frequency transform is applied at the encoder apparatus, the decoder apparatus may be arranged to select a suitable predetermined frequency domain decoding operation rather than e.g., a nominal time domain decoding operation thereby matching the encoder apparatus operation.

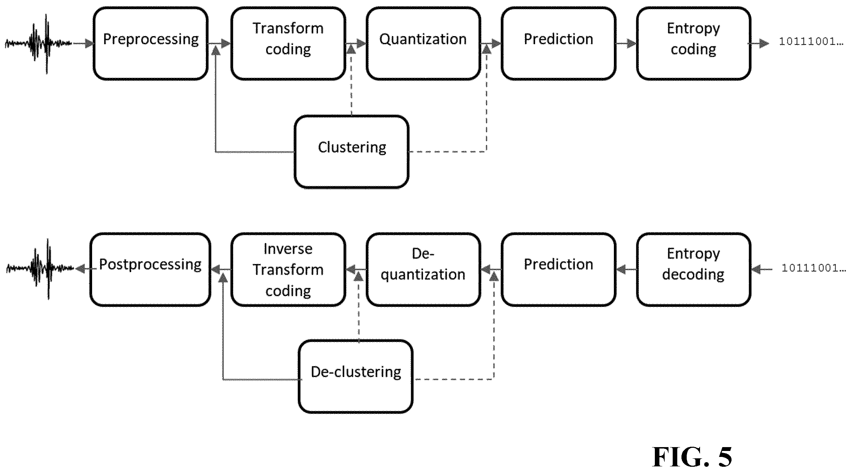

The processing of the encoding apparatus may include applying a sequence of operations to the biomedical signals to generate the encoded compressed data. As illustrated in FIG. 5, the encoding operation may for example include a pre-processing operation (e.g., scaling/normalization, z-score transform/standardization, log transform, Principal Component Analysis (PCA), Independent Component Analysis (ICA), Linear Discriminant Analysis (LDA), Singular Value Decomposition (SVD) ), a signal transform (e.g., a time to frequency transform (DCT, QMF, FFT, DWT etc.), a quantization operation (typically a compression by mapping the biomedical signal into coarser quantized versions, typically with the quantization being variable for different frequency domains, with time, and/or as a function of the signal properties), followed by a relative/predictive coding, and finally an entropy coding of the resulting data.

The processing at the decoder apparatus may, as also illustrated in FIG. 5, perform the inverse/complementary operations in the reverse order to recover/replicate the original biomedical signals. Thus, specifically the decoder apparatus may first perform entropy decoding followed by the relative/predictive decoding. The decoded signals are then de-quantized and thus compensated for the quantization performed at the encoder. A signal transform which is the inverse of the one performed at the audio encoder may then be applied followed by a post-processing which performs the inverse operation of the pre-processing performed by the encoder apparatus.

In such cases, where a sequence of operations is applied as part of the encoding (and decoding) process, the clustering may be performed at different positions (and on different signals) of the sequence. For example, as indicated in FIG. 5, the clustering process may e.g., be based on the biomedical signals after pre-processing (it could in principle also be performed on the biomedical signals prior to the pre-processing), after the signal transform, or after quantization. Depending on the specific properties and preferences of the individual embodiment, the clustering may be advantageous at the different positions, or indeed considering the signals from multiple positions may be advantageous. Accordingly, the encoder may adapt the signals that are used for the clustering, and thus the positioning of the clustering.

In some embodiments, the clustering may thus be different for different encoder apparatuses, or in some embodiments, a given encoder apparatus may be arranged to dynamically modify and change the position of the clustering.

The data signal generator 105 may be arranged to include an indication of the position of the clustering in the sequence. The data signal generator 105 may accordingly add metadata which indicates on the basis of which signals of the sequence the clustering is performed. For example, a single data value or index may be included which indicates the block/operation from which the biomedical signals are provided that are then clustered.

This may allow the decoder apparatus to adapt its operation to match that of the encoder apparatus. It may accordingly allow efficient adaptation of the encoding and decoding processes to allow improved operation. In particular, it more effectively exploits data characteristics at each stage to enhance computational efficiency, minimize distortion and improve compression.

In some embodiments, the clustering may further be dependent on a position associated with the (acquisition of the) biomedical signals. The position may specifically be a position on the test subject/patient. The positions may be provided in any suitable way, such as metadata together with the biomedical signals, as user inputs, as predetermined associations between specific signals/sensors and positions etc.

In such a case, the position information may e.g., be used as another similarity indication for the biomedical signals, such as e.g., by considering closer positions to be more similar. In other cases, the positions may be used as constraints such as e.g., by only allowing clustering of biomedical signals for which the positions are at a distance below a threshold.

For example, in some biomedical applications, sensors at certain positions could be placed in predetermined groups, which are typically desired to be processed together. The proximity to other sensors, or e.g., to a nominal position, could be considered as part of the clustering.

Such a clustering may provide improved performance in many scenarios.

In particular, considering position information in the clustering process can not only greatly reduce the search-space for determining appropriate clusters (thereby decreasing computational complexity at the encoder side, in particular for biomedical signals comprising a large number of channels), but is also likely to reduce the amount of metadata for coding the cluster information. Providing limitations on the channels that can appear in a cluster based on position may result in channel indices (within a cluster) that are close to each other, which can be efficiently exploited by differentially coding these differences.

In many embodiments, the data signal generator 105 may generate metadata that is added to the biomedical data signal to indicate to the decoding apparatus the clustering method that has been used, and/or how channels have been allocated to clusters. The metadata may further include an indication of positions for biomedical signals thereby e.g., allowing the decoder apparatus to replicate the process.

In some embodiments, the data encoder 103 may be arranged to apply different encoding processes/algorithms/standards. For example, the data encoder 103 may be arranged to switch between a high loss relative encoding, a low loss relative encoding, and a non-relative encoding. The data encoder 103 may in some such cases be arranged to perform a dynamic selection of which encoding algorithm to use dependent on e.g., signal properties, user inputs or other parameters.

A particular advantage of the described approach is that in many embodiments the data encoder 103 may be arranged to individually select an encoding algorithm for different clusters. Thus, in many cases at least two of the clusters may (in some time intervals) be encoded using different encoding processes. Indeed, the data encoder 103 may in many embodiments be arranged to individually select an encoding algorithm for each cluster.

For example, some encoding algorithms are highly suitable for encoding transient (fast changing) effects whereas other algorithms are highly suitable for encoding more static signals. The clustering may typically result in signals with high transient components being clustered together and with more static signals being clustered together. The data encoder 103 may accordingly proceed to evaluate e.g., average or median signal properties for each cluster and select the most appropriate algorithm for the specific cluster. In many embodiments, the data encoder 103 may advantageously evaluate signal spectrum properties and/or a variation over time may be evaluated. Such a selection may include selecting a non-relative/predictive encoding for a cluster which may have been generated to include biomedical signals that are not highly similar (such as a residual cluster comprising biomedical signals that are not being included in any other cluster by the clustering process).

In cases where different encoding algorithms may be selected, the data signal generator 105 may be arranged to include metadata in the biomedical data signal which indicates the algorithms that have been selected for the clusters. The decoder apparatus may then apply the corresponding decoding process.

In some embodiments, the data encoder 103 may be arranged to time align the biomedical signals within a cluster. In some cases, time alignment may be applied to all clusters whereas in other approaches it may only be applied to a subset.

The time alignment may time shift/offset one or more of the biomedical signals of a given cluster. The time alignment may typically be such that it results in a reduced cross correlation between the signals. The time alignment may typically be variable. For example, a reference biomedical signal may be selected within each cluster (e.g., randomly or the one closest to a centroid of the cluster etc.). The data encoder 103 may then proceed to determine a cross correlation as a function of time for each of the other biomedical signals in the cluster. Each of the biomedical signals may then be time offset/shifted by an amount corresponding to the time offset for which the determined cross correlation is maximum. As a result, the biomedical signals of the cluster may be time shifted to be more correlated thereby allowing an improved relative encoding.

In such embodiments, the data encoder 103 may provide information of the applied time shifts to the data signal generator 105 which may proceed to include metadata describing the time shifts. The decoder apparatus may proceed to decode the biomedical signals and then apply the time shifts/offsets as indicated in the metadata.

Thus, in some instances, it may be beneficial to time-align the biomedical signals within a cluster. In this instance, after the clusters are determined, a measure, such as the cross-correlation between the biomedical signals, can be used to determine a time-shift that can be applied to one or more of the biomedical signals within the cluster to increase the correlation between those biomedical signals. This time shift would be communicated to the decoder in the bitstream metadata to enable the inverse correction during decoding.

The combination of the time alignment and the clustering provides an extremely efficient encoding process that in many scenarios may provide a substantially reduced data rate for a given signal quality of the decoded biomedical signals.

In some embodiments, rather than directly encode and represent the received biomedical signals, the encoder apparatus may perform a re-referencing on the biomedical signals such that compensated biomedical signals are generated which represent the signals with respect to (at least one) reference signal being derived from the received multichannel biomedical signal. Thus, rather than the biomedical signals being e.g., differential voltage signals with respect to a second electrode, the biomedical signals are re-referenced to be relative to a reference signal generated by the encoder apparatus from the multichannel biomedical signal.

Such approaches may be particularly advantageous for scenarios wherein each of the biomedical signals represent a measured differential voltage between two measurement electrodes and the following description will focus on such examples.

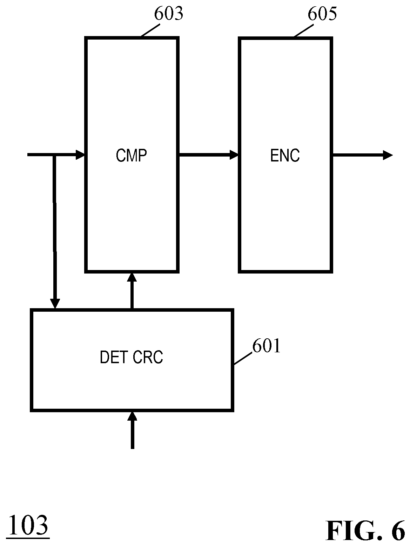

As illustrated in FIG. 6, the data encoder 103 may include circuitry for re-referencing the biomedical signals before encoding. The re-referencing may be based on the clustering process such that re-referencing is performed for biomedical signals within the same cluster.

In the example, the biomedical signals are fed to a determination circuit 601 which is arranged to determine a reference signal from the plurality of biomedical signals. Specifically, for one or more clusters, a reference signal is determined from the biomedical signals belonging to the cluster. As a specific example, the reference signal may be generated as the average signal for all the biomedical signals in the cluster, i.e., for a given time instant, the reference signal may be determined as the average value of all the biomedical signal values. It will be appreciated that a number of different approaches may be applied to determine the reference signal depending on the preferences and requirements for the individual embodiment and application as will be described in more detail later.

In most embodiments, the reference signal may be selected from the same cluster as the signals being compensated but it is possible in some embodiments for the reference signal to belong to another cluster. A cluster comprising all the reference signals could even be envisaged.

The determination circuit 601 is coupled to a compensation circuit 603 that generates compensated biomedical signals by compensating at least some, and often all, of the biomedical signals of the cluster for the reference signal. In many embodiments, the compensation circuit 603 may be arranged to subtract the reference signal from the biomedical signals being compensated, and thus the compensated signals may be differential voltage signals relative to a (potentially virtual) reference voltage potential represented by the reference signal. The compensated biomedical signals may thus be signals corresponding to the input biomedical signals but re-referenced with respect to the generated reference signal.

The compensation circuit 603 is coupled to an encoder circuit 605 which is arranged to encode the compensated biomedical signals, e.g. as previously described for the data encoder 103. Thus, the encoder circuit 605 generates compressed encoded data for the compensated biomedical signals.

The decoder apparatus may, when decoding the biomedical data signal, perform the reverse compensation operation, i.e., it may determine the reference signal and reverse the compensation.

Specifically, the decoder apparatus may generate intermediate biomedical signals by decoding the compressed encoded data of the biomedical data signal. It may then perform the complementary operation to the encoding of the encoder apparatus and may thus generate local replicas of the compensated biomedical signals. Thus, the intermediate biomedical signals may be recreations of the compensated biomedical signals.

The decoder apparatus may determine the reference signal from the biomedical data signal. In many cases, the reference signal may be explicitly encoded in the biomedical data signal and the decoder apparatus may simply decode the received encoding data for the reference signal.

The decoder apparatus may then generate biomedical signals by (de)compensating the intermediate biomedical channels by the reference signal. It may specifically be arranged to perform the complementary/reverse operation to that performed by the compensation circuit 603 of the encoder apparatus. The decoder apparatus may accordingly reverse the operation/signal processing/effect of the re-referencing performed at the encoder apparatus. In particular, in many embodiments, the decoder apparatus is arranged to add the reference signal to each of the decoded intermediate channel signals.

The decoder apparatus may accordingly modify the received intermediate channel signals to compensate for the re-referencing performed by the encoder apparatus. It generates biomedical signals that are local replicas/estimates of the biomedical signals of the multichannel biomedical signal input to the encoder apparatus.

The approach may provide a highly advantageous coding (and typically compression) of a multichannel biomedical signal. An improved quality/accuracy to data rate trade-off can be achieved in many situations and for many scenarios and applications. Further, the approach can be implemented with low complexity and/or computational resource requirements.

In more detail, e.g., amplifiers for bio-electrical signal measurements, such as electroencephalography (EEG), electrocardiography (ECG), and electromyography (EMG), measure voltages relative to reference and ground electrodes, in order to provide stable and meaningful captures and recordings. The reference electrode serves as the baseline voltage against which the active electrodes' signals are measured and further a ground electrode helps reduce electrical noise and interference by providing a common return path for the amplifier. The choice of reference electrode is often selected as a position/location where no bioelectrical activity of interest is measured, and the ideal position varies depending on the signal type. For EEG, common references include the mastoid, linked ears, or a centrally located electrode (e.g., Cz in the 10-20 system). For ECG, a single limb electrode (e.g., right leg) or Wilson's central terminal (averaged limb electrodes) is often used. For EMG, a nearby electrically neutral site is often preferred. However, despite seeking to optimize positions for reference electrodes, the reference is not always ideal for all electrodes or conditions.

Re-referencing is performed to improve signal quality, reduce common noise, and enhance spatial resolution. One re-referencing method is to use the average reference, where the mean signal across all electrodes is subtracted from each biomedical signal to reduce common noise. Another method is to use the linked mastoid reference, which averages signals from two mastoid electrodes to create a more stable reference. The common average reference (CAR) may be used e.g., in high-density EEG and intracranial recordings, helping to minimize spatially widespread noise.

In some embodiments, the encoding of a plurality of biomedical signal includes a compensation/re-referencing based on a reference signal generated from the biomedical signals.

Such re-referencing can help reduce entropy in the biomedical signal measurements by reducing noise and artifacts that contribute to randomness. E.g., by aligning the biomedical signals to a more stable baseline, re-referencing can improve data consistency, potentially making it more predictable and compressible.

In the described approach, a compression ratio for a lossless and / or lossy compression of the biomedical signals may be substantially increased. This is achieved by re-referencing each (or at least some) of the biomedical channels to one or more reference channels. This is specifically done such that the variance between the biomedical signals is minimized. This is of particular use for EEG signals, where there is potentially a high number of channels, but may also be useful for other signal types such as e.g., ECG. In some embodiments, a reference channel that all other channels are re-referenced to may be one of the already existing channels, while in other embodiments it may be a new channel that is derived from the existing channels, such as the Common Average Reference (CAR). In the latter case the number of channels is increased, but due to the reduced variation between the other channels, greater compression ratios may be achieved. Typically, re-referencing may be applied to the entire multichannel biomedical signal, but in some cases it may only be applied to a subset of biomedical signals or in clusters. In some cases, the compression/encoding may be performed in frames and in such cases the described operation, and specifically the re-referencing, may be applied on a frame-by-frame basis.

In many embodiments, the data signal generator 105 is arranged to include metadata in the biomedical data signal which describes one or more properties of the compensation of the biomedical signals. The data signal generator 105 may be arranged to include a compensation indication in the biomedical data signal where the compensation indication is indicative of a property of the compensation performed by the compensation circuit 603.

In some embodiments, the compensation indication may be a single data bit flag, such as e.g., an indication whether the compensation has been performed or not. The compensation indication may in some embodiments be a flag that indicates whether the compressed encoded data represents compensated biomedical signals or non-compensated biomedical signals. Such an approach may be suitable e.g., for situations where the compensation may be a predetermined operation/compensation. For example, the encoder apparatus may be arranged to derive a reference signal from the biomedical signals and to include the resulting reference signal in the biomedical data signal, or e.g., if the reference signal is also a biomedical signal, it may include a reference signal simply be indicating that one of the included signals is a reference signal. The decoder apparatus may then be arranged to decode the compressed encoded data to recreate the biomedical signals. If the compensation indication indicates that compensation has been applied, it may further proceed to recreate the reference signal and combine this with (typically add to) the biomedical signals. If the compensation indication indicates that no compensation has been performed, the decoded biomedical signals may be used directly without any compensation.

In other embodiments, a compensation indication may be included which provides substantial information on the compensation that has been performed. For example, the compensation indication may include data describing how the compensation has been performed (e.g., whether a straight subtraction has been performed or whether more complex operations have been performed such as e.g., whether filtering, weighting, etc., has been included in the compensation). In some embodiments, the compensation indication may include data that indicates how the reference signal has been generated, such as e.g., whether it has been selected as one of the biomedical signals, is a combination of biomedical signals, etc.

The data signal generator 105 may in some embodiments be arranged to include a compensation indication that provides an indication of a function used to derive the reference signal.

In some embodiments, the compensation indication may provide a direct indication of the reference signal, such as for example by indicating that one of the received biomedical signals is to be used as a reference signal.

Indeed, in some embodiments, compressed encoded data that represents an encoding of a reference signal may directly be considered a compensation indication e.g., if it is also used as an indication that compensation has been performed. For example, if a dedicated encoded reference signal is included in the biomedical data signal, the decoder apparatus may interpret this as an indication that compensation has been performed and it may accordingly proceed to decode the reference signal and e.g., add this to the received encoded biomedical signals.

As an example, the compensation indication may provide an indication of one of the biomedical signals (e.g., a channel index indicator). The compensation indication may further e.g., optionally provide information on e.g., a gain factor with which the indicated channel should be scaled. As another example, the compensation indication could indicate multiple biomedical signals (e.g., multiple channel indices) along with linear combination weights that should be applied to these in order for a local linear combination to generate the desired reference signal. As another example, the compensation indication may indicate that the reference signal is a separate reference signal that is derived from (but not equal to any of) the biomedical signals. In this case, the indicator could be a channel index of the reference channel with an indication that the reference channel is not one of biomedical signals, but an extra channel.

The exact approach or algorithm for deriving the reference signal from the biomedical signals will depend on the preferences and requirements of the individual embodiment.

In many embodiments, the determination circuit 601 may select one of the biomedical signals as the reference signal.

In other embodiments, the determination circuit 601 may be arranged to derive the reference signal from the biomedical signals, and specifically the reference signal may be generated as a combination of two or more, and indeed typically all, of the biomedical signals.

In many embodiments, the reference signal may be generated such that a variation and/or level/amplitude of the compensated signals is reduced, and preferably minimized. In particular, in many embodiments, the reference signal may be generated such that a combined entropy of the compensated biomedical channels signals is reduced, and preferably minimized.

In the context of signal compression and more generally information theory, entropy measures the amount of unpredictability or randomness in the data. Lower entropy means the data is more predictable and thus easier to encode and compress effectively.

In some embodiments, the determination circuit 601 may generate and evaluate a potentially large number of reference signals using different approaches and may for each signal generate compensated biomedical signals. It may then evaluate a variation/level/entropy measure for each compensated biomedical signal of a given set/cluster of biomedical signals that are to be coded relative to the reference signal. It may then generate a combined variation/level/entropy measure for the set/cluster of biomedical signals and proceed to select the reference signals as the signal that results in the lowest variation/level/entropy measure.

In some embodiments, the determination circuit 601 may, thus, generate the reference signal as a signal that reduces a difference measure which is indicative of a difference between the first reference signal and a set of the biomedical signals.

As another example, in some embodiments, the determination circuit 601 may comprise a trained neural network which as input receives the biomedical signals, or feature values that represent properties thereof, and may provide a reference signal as an output. Such a trained neural network may have been trained using a large sample of multichannel biomedical signals and using a range of different approaches for generating reference signals (e.g., including manual generation by human intervention) and using a loss function which is dependent on a variation/level/entropy measure generated for biomedical signals that have been compensated using the reference signal generated for the sample multichannel biomedical signal.

In many embodiments, the determination circuit 601 may be arranged to directly generate a reference signal from the multichannel biomedical signal without performing any compensation operation or evaluating any properties of resulting compensated biomedical signals. Rather, in some embodiments, the reference signal may be derived directly by applying a suitable function or algorithm to the biomedical signals of the multichannel biomedical signal.

In some embodiments, for example, the determination circuit 601 may be arranged to reduce the power/variance of differences between the biomedical signals and the reference signal. This may specifically be achieved by averaging the biomedical signals, and specifically the reference signal may be generated as the average signal value of the biomedical signals, such as specifically the Common Average Reference (CAR).

In some embodiments where the reference signal is selected as one of the biomedical signals of the multichannel biomedical signal, the reference signal may for example be selected as the one for which the combined difference to the other biomedical signals (for which the reference signal is to be applied) is minimized. The biomedical signal may be selected as the one with the highest combined correlation to the other biomedical signals.

In some embodiments, the determination circuit 601 may directly determine the reference signal by selecting between a set of candidates, such as specifically the set of candidates consisting in the biomedical signals of the multichannel biomedical signal, based on a property of the signals.

For example, in some embodiments, the determination circuit 601 may determine the reference signal as the candidate signal for which a variation measure is minimized/the lowest. The variation measure may specifically be a variance, a standard deviation, or indeed an entropy measure.

In some embodiments, the determination circuit 601 may be based on a relative measure between the candidate biomedical signals. For example, in some embodiments, the determination circuit 601 may determine the reference signal as the biomedical signal having the highest combined correlation with the other biomedical signals.

The determination of the determination circuit 601 may be an automatic process based on factors such as signal type, number of channels, variance between channels, etc. The reference signal may be determined in different ways and in many cases different candidate options may be considered. For example, initially the CAR may be generated (as the sum/average of all biomedical signals). The reference signal may then e.g., be selected from the group comprising the CAR signal and the biomedical signals of the multichannel biomedical signal. The reference signal may for example be selected as the biomedical signals of:
- the channel with the highest correlation with all other channels,
- the channel with the lowest standard deviation,
- the channel with the lowest entropy,
- the channel which, after compensation, gives the lowest overall standard deviation across all channels, or
- the channel which, after compensation, gives the lowest overall entropy across all channels.

The evaluation of the variation/level/entropy may include the lossy or lossless compression stage.

In some embodiments, the encoder apparatus may as described proceed to include encoded reference signal data that describes the reference signal(s). In such cases, the decoder apparatus may directly decode the encoded reference signal data and then proceed to apply the generated reference signal to the received biomedical signals. It will be appreciated that in such cases, any suitable encoding and compression approach may be used for the reference signal. Typically, the same approach may be used for the reference signal and for each of the compensated biomedical signals. Indeed, in some cases, the reference signal may be a non-compensated biomedical signal of the multichannel biomedical signal.

In many cases, a single reference signal may be generated, and this may be used to compensate all (other) biomedical signals. However, in some embodiments, the compensation circuit 603 may be arranged to apply the compensation based on a given reference signal to only a subset of the biomedical signals. For example, the reference signal may only be applied to compensate biomedical signals that correspond to measurement electrodes close to an electrode used to generate the reference signal or may e.g., only be applied to biomedical signals that are highly correlated with the reference signal.

In some embodiments, a given reference signal may accordingly only be applied to a subset of biomedical signals and the multichannel biomedical signal may include data for other biomedical signals. The subset of biomedical signals may be determined as one of the clusters generated by the clustering process. In some such embodiments, these remaining biomedical signals may be included without any compensation. However, in many embodiments, the determination circuit 601 may be arranged to generate a plurality of reference signals and different biomedical signals may be encoded relative to different reference signals. Thus, a first subset/cluster of biomedical signals may be compensated using one reference signal and a second subset/cluster of biomedical signals, typically disjoint with the first subset/cluster of biomedical signals, may be compensated using a different reference signal.

In cases where multiple reference signals are generated, these may be derived from different subsets of biomedical signals. In particular, in many embodiments, the biomedical signals of the multichannel biomedical signal may be divided into different subsets and a reference signal may be generated for each individual subset. The reference signal may in such cases typically be used to compensate the biomedical signals for that subset. Thus, in many embodiments, the subset used for generating a reference signal and the subset for which the reference signal is used for compensation are identical. However, in some embodiments, the set of biomedical signals used for deriving a reference signal and the set of biomedical signals to which the reference signal is applied may be different.

As an example, in some embodiments, the received biomedical signals may be divided into a plurality of groups/subsets/clusters. The division may in some embodiments be based on position information and the subsets may e.g., be determined as groups of biomedical signals for measurement electrodes with a maximum distance between any two biomedical signals not exceeding a threshold. Alternatively, or additionally, the grouping into subsets may be based on the signal properties of the biomedical signals. For example, the cross-correlation between all possible pairs of biomedical signals may be determined and the biomedical signals may subsequently be grouped into e.g., a predetermined number of groups such that the lowest correlation between pairs of the same group is maximized. It will be appreciated that many algorithms are known and can be used for grouping, including more complex approaches such as e.g., using a clustering algorithm.

The determination circuit 601 may then proceed to derive a reference signal for each of the subsets, such as e.g., determining a CAR or using any of the previously described examples. The determined reference signal for each subset is then used to compensate the biomedical signals of that subset, such as typically by generating the compensated biomedical signals as difference signals relative to the reference signal. The resulting compensated biomedical signals may then be encoded as previously described.

Thus, in some embodiments, re-referencing may be applied to all biomedical signals of the multichannel biomedical signal, or may in some embodiments be applied to subsets of biomedical signals independently. In the latter case, there may be multiple reference signals with each of these being used to re-reference one or more subsets of the biomedical signals.

Thus, the re-referencing may happen on subsets of the biomedical signals, in which case for each subset, a reference signal can be derived e.g., as
- A CAR for the subset
- A biomedical signal of the subset with the lowest/highest standard deviation
- The biomedical signal of the subset with the lowest/highest entropy,
- The biomedical signal of the subset with the lowest/highest correlation
- A biomedical signal, or a signal derived therefrom, of the subset which after compensation results in compensated biomedical signals for the subset with a lowest variation measure, such as a lowest standard deviation, variance, or entropy.

The data signal generator 105 may be arranged to generate metadata that describes the subset operation. Specifically, the data signal generator 105 is arranged to include an indication of the division into subsets. In particular, it may provide an indication of the biomedical signals that are comprised in the individual subset. It may specifically provide such an indication for all subsets of the biomedical signals which have been differently compensated.

In particular, the data signal generator 105 may include an indication of each subset. It may further for each subset provide an indication of the reference signal and/or of a property of the algorithm that has been used to generate the reference signal. Specifically, the data signal generator 105 may proceed to include information corresponding to the compensation indication previously described, but with this information being specific to a given subset. This may be performed for each subset, and the data signal generator 105 may proceed to generate metadata that includes an indication of each subset and a compensation indication for each subset. The compensation indication may in many embodiments directly indicate the reference signal for the subset.

For example, the data signal generator 105 may proceed to generate metadata which for each subset first lists channel indices for all the biomedical signals in the subset followed by an indication of a channel index for a reference signal that is used to compensate that group.

In many embodiments, the reference signals for a plurality of subsets may be encoded and included in the biomedical data signal. The biomedical data signal may accordingly include compressed encoded data for a plurality of subsets. In such cases, the encoding of one reference signal may be relative to another reference signal. For example, a first reference signal may be fully encoded using a suitable encoding format as previously described. The encoder apparatus may further be arranged to decode this signal to generate a decoded version of the first reference signal. A second reference signal for a different group may then be compensated for the decoded first reference signal, and specifically the decoded first reference signal may be subtracted from the second reference signal. The resulting compensated/difference/relative signal may then be encoded using a suitable encoding algorithm and the resulting encoded data may be added to the biomedical data signal.

In such cases, the data signal generator 105 may further include metadata that includes an indication that the second reference signal is encoded relative to the first reference signal. The decoder apparatus may, when parsing the data signal, determine that two reference signals are present and may in response to the indication that the second reference signal is encoded relative to the first reference signal, proceed to first decode the first reference signal and the difference second reference signal. It may then generate the second reference signal by combining the first reference signal and the difference second reference signal, typically simply by adding these together.

In cases where the biomedical data signal includes three reference signals, the third reference signal may be encoded relative to both the first and second reference signals. For example, the first reference signal and the difference second reference signal may be subtracted from the third reference signal and the resulting signal may be encoded. As another example, a linear combination of the first and second reference signals may be subtracted from the third reference signal and the remaining reference signal may be encoded. In such cases, weights of the linear combination may be determined to minimize the level/entropy of the compensated third reference signal. In such cases, the data signal generator 105 may generate the metadata to include an indication that the third reference signal is encoded relative to the first and second reference signals. In the example of the linear combination, the metadata may further include data indicating the weights of each of the first and second reference signals.

For some cases the re-referencing may happen in pairs or small clusters that are spatially located close to each other. This may be determined at the encoder, or be a pre-determined set of pairings/groupings. Referencing to neighboring channels has the benefit of removing noise from and spatially diffuse artifacts.

In some embodiments, the receiver 101 may further receive measurement position data for at least some of the biomedical signals. The measurement positions may typically be indications of a position on a body of the subject at which the electrode for the measurement is placed. The position information may be for relative positions with respect to positions for other channels. In some embodiments, the position information may be manually input. In some embodiments, measurement positions for the electrodes generating the biomedical signals may be predetermined. For example, for EEG or ECG, each electrode may be predetermined and marked for measurement at a particular position on the subject and the measurement is based on an assumption that the operator places the electrode at the correct position. In yet other embodiments, the encoder apparatus may be arranged to determine the position data based on the signal properties. For example, for an ECG application, and assuming the electrodes are positioned at predetermined nominal measurement positions, the biomedical signals may be compared to expected signals for these positions, and it can be determined which biomedical signal corresponds to which nominal measurement position.

In some embodiments, the determination circuit 601 is arranged to take the measurement positions into account when determining the reference signal(s).

For example, in some embodiments, the determination circuit 601 may determine the reference signal as the biomedical signal which is closest to the center of the measurement positions. In some embodiments, particular measurements may be suitable for providing a reference due to their particular position on the body, and the reference signal may be determined as the biomedical signal for the electrode closest to this position. In some embodiments, particularly measurement positions may be expected to essentially provide the same measurement result and the biomedical signals closest to such position may be selected and used to derive the reference signal (e.g., as the average thereof). In some embodiments, the reference signal may be determined as a linear combination of the surrounding electrodes of the biomedical signal(s).

In many cases, the position information may be used to group the biomedical signals into subsets. For example, as previously described, based on the position information, the biomedical signals may be grouped into subsets comprising biomedical signals for measurements that are in close proximity.

The compensation approach may allow advantageous generation of a biomedical data signal representing a plurality of biomedical signals. It may in many embodiments allow an improved data rate to quality trade-off, and may in many embodiments provide advantageous compression/encoding of the multichannel biomedical signal. The approach may allow a reduced bandwidth/data rate for compressed encoded data representing a multichannel biomedical signal. In many embodiments, the storage requirements and/or required communication bandwidth may be reduced substantially. The approach may allow efficient and practical implementation and/or may reduce complexity and processing/computational requirements.

As previously mentioned, the biomedical waveform signal may for example be an electrocardiography (ECG), electroencephalography (EEG), or electromyography (EMG) signal but any other bioelectrical signals, such as, but not limited to, Electrocorticogram (ECoG), Electrooculogram (EOG), Electroretinogram (ERG), Fetal electrocardiography (fECG), Electrohysterogram (EHG), Electrodermal Activity (EDA), or Electrogastrogram (EGG) could also apply.

Generating the encoded compressed data may include applying a data compression algorithm to the compensated biomedical signals. The data encoder may be arranged to apply a data compression algorithm to the compensated biomedical signals.

In some embodiments, a plurality, and possibly all, biomedical signals represent a measured differential voltage between a common reference electrode and a measurement electrode for the biomedical signal. In some embodiments, a first biomedical signal represents a measured differential voltage between a first pair of electrodes and a second biomedical signal represents a measured differential voltage between a second pair of electrodes, the first pair of electrodes not including any electrode of the second pair of electrodes.

In some embodiments, the data signal generator is arranged to include a compensation indication in the biomedical data signal, the compensation indication being indictive of a property of the compensation of the at least some of the plurality of biomedical channels signals.

This may provide particularly advantageous operation and/or performance in many embodiments and scenarios.

The compensation indication may be included as metadata. The compensation indication may be indicative of whether the compensation has been performed or not. The compensation indication may be indicative of how the compensation has been performed and/or of how the reference signal has been determined. In many embodiments, the compensation indication may provide an indication of a property of the derivation of the reference signal. In some embodiments, the compensation indication may be indicative of a function used to determine the reference signal.

In some embodiments, the data encoder is arranged to generate encoded reference signal data for the first reference signal; and the data signal generator is arranged to generate the biomedical data signal to comprise the encoded reference signal data.

This may provide particularly advantageous operation and/or performance in many embodiments and scenarios.

In some embodiments, the determination circuit is arranged to select a biomedical signal of the plurality of biomedical signals as the first reference signal.

This may provide particularly advantageous operation and/or performance in many embodiments and scenarios. It may typically provide reduced complexity and/or a reduced data rate.

In some embodiments, the determination circuit is arranged to generate the first reference signal as a combination of at least some of the biomedical signals.

This may provide particularly advantageous operation and/or performance in many embodiments and scenarios. It may often provide an improved first reference signal allowing a reduced encoded data rate for the biomedical signals. The combination may be a linear combination, and specifically may be an averaging/summation.

In some embodiments, the determination circuit is arranged to generate the first reference signal as a signal that reduces a difference measure, the difference measure being indicative of a difference between the first reference signal and a set of signals from the plurality of biomedical signals.

This may provide particularly advantageous operation and/or performance in many embodiments and scenarios.

In some embodiments, the determination circuit may be arranged to generate the first reference signal as a signal for which a difference measure indicative of a difference between the first reference signal and a set of biomedical signals is minimized/optimized.

In some embodiments, the determination circuit may be arranged to generate the first reference signal as a signal for which a statistical measure indicative of a difference between the first reference signal and a set of biomedical signals is minimized/optimized.

In some embodiments, the determination circuit is arranged to determine the first reference signal as a biomedical signal having a highest combined correlation with the other biomedical signals.

In some embodiments, the compensation circuit is arranged to determine the at least some of the plurality of biomedical channels signals as a first subset of biomedical signals.

This may provide particularly advantageous operation and/or performance in many embodiments and scenarios.

In many embodiments, the determination circuit is arranged to determine the first reference signal from only biomedical signals comprised in the first subset of the biomedical signals.

In some embodiments, the data signal generator is arranged to include an indication of the first subset in the biomedical data signal.

This may provide particularly advantageous operation and/or performance in many embodiments and scenarios.

In some embodiments, the determination circuit is arranged to derive a second reference signal from the plurality of biomedical signals; and the compensation circuit is arranged to generate further compensated biomedical signals by compensating a second set of biomedical signals by the second reference signal; the data encoder is arranged to generate further encoded compressed data for the further compensated biomedical signals; and the data signal generator is arranged to generate the biomedical data signal to comprise the further compressed encoded data.

This may provide particularly advantageous operation and/or performance in many embodiments and scenarios.

In many embodiments, the determination circuit is arranged to determine the second reference signal from only biomedical signals comprised in the second subset of the biomedical signals.

In some embodiments, the compensation circuit is arranged to generate at least one compensated biomedical signal by compensating a biomedical signal by a combination of the first reference signal and the second reference signal; the data encoder is arranged to generate encoded compressed data for the compensated biomedical signal; and the data signal generator is arranged to generate the biomedical data signal to comprise this compressed encoded data.

In some embodiments, the data signal generator is arranged to include an indication of the second subset in the biomedical data signal.

This may provide particularly advantageous operation and/or performance in many embodiments and scenarios.

In some embodiments, the data encoder is arranged to generate further encoded compressed data by encoding the second reference signal relative to the first reference signal.

This may provide particularly advantageous operation and/or performance in many embodiments and scenarios.

In some embodiments, the data signal generator is arranged to include an indication indicative of the second reference signal being encoded relative to the first reference signal.

This may provide particularly advantageous operation and/or performance in many embodiments and scenarios.

In some embodiments, the determination circuit is arranged to determine a third reference signal from the plurality of biomedical signals; and the compensation circuit is arranged to generate further compensated biomedical signals by compensating a third set of biomedical signals by the third reference signal; the data encoder is arranged to generate further encoded compressed data for these further compensated biomedical signals; and the data signal generator arranged to generate the biomedical data signal to comprise this further compressed encoded data.

The encoder may be arranged to generate further encoded compressed data by encoding the third reference signal relative to a combination of the first reference signal and the second reference signal.

In some embodiments, the determination circuit is arranged to determine the first reference signal in dependence on measurement positions for the measurement electrodes.

This may provide particularly advantageous operation and/or performance in many embodiments and scenarios.

The apparatus(es) may specifically be implemented in one or more suitably programmed processors. The different functional blocks may be implemented in separate processors and/or may e.g., be implemented in the same processor. An example of a suitable processor is provided in the following.

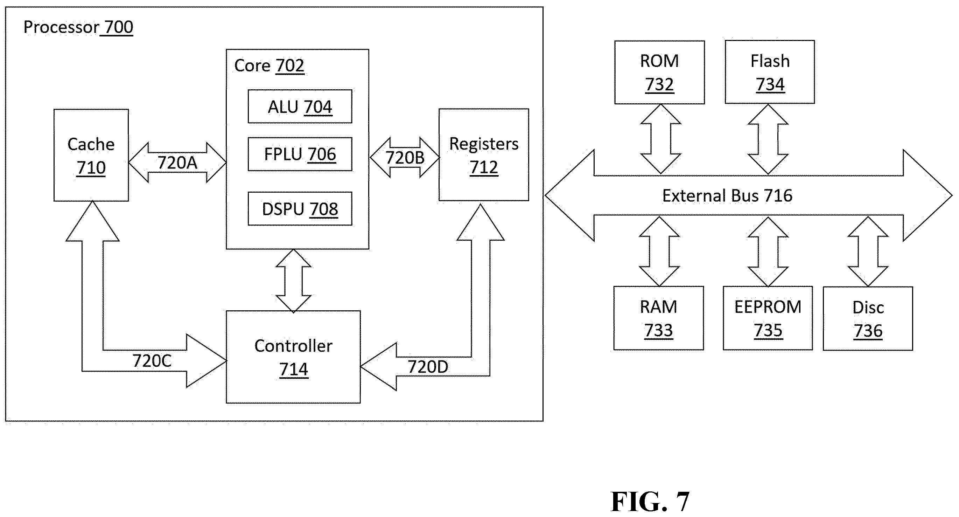

FIG. 7 is a block diagram illustrating an example processor 700 according to embodiments of the disclosure. Processor 700 may be used to implement one or more processors implementing an apparatus as previously described or elements thereof. Processor 700 may be any suitable processor type including, but not limited to, a microprocessor, a microcontroller, a Digital Signal Processor (DSP), a Field ProGrammable Array (FPGA) where the FPGA has been programmed to form a processor, a Graphical Processing Unit (GPU), an Application Specific Integrated Circuit (ASIC) where the ASIC has been designed to form a processor, or a combination thereof.

The processor 700 may include one or more cores 702. The core 702 may include one or more Arithmetic Logic Units (ALU) 704. In some embodiments, the core 702 may include a Floating Point Logic Unit (FPLU) 706 and/or a Digital Signal Processing Unit (DSPU) 708 in addition to or instead of the ALU 704.

The processor 700 may include one or more registers 712 communicatively coupled to the core 702. The registers 712 may be implemented using dedicated logic gate circuits (e.g., flip-flops) and/or any memory technology. In some embodiments the registers 712 may be implemented using static memory. The registers may provide data, instructions and addresses to the core 702.

In some embodiments, processor 700 may include one or more levels of cache memory 710 communicatively coupled to the core 702. The cache memory 710 may provide computer-readable instructions to the core 702 for execution. The cache memory 710 may provide data for processing by the core 702. In some embodiments, the computer-readable instructions may have been provided to the cache memory 710 by a local memory, for example, local memory attached to the external bus 716. The cache memory 710 may be implemented with any suitable cache memory type, for example, Metal-Oxide Semiconductor (MOS) memory such as Static Random Access Memory (SRAM), Dynamic Random Access Memory (DRAM), and/or any other suitable memory technology.

The processor 700 may include a controller 714, which may control input to the processor 700 from other processors and/or components included in a system and/or outputs from the processor 700 to other processors and/or components included in the system. Controller 714 may control the data paths in the ALU 704, FPLU 706 and/or DSPU 708. Controller 714 may be implemented as one or more state machines, data paths and/or dedicated control logic. The gates of controller 714 may be implemented as standalone gates, FPGA, ASIC or any other suitable technology.

The registers 712 and the cache 710 may communicate with controller 714 and core 702 via internal connections 720A, 720B, 720C and 720D. Internal connections may be implemented as a bus, multiplexer, crossbar switch, and/or any other suitable connection technology.

Inputs and outputs for the processor 700 may be provided via a bus 716, which may include one or more conductive lines. The bus 716 may be communicatively coupled to one or more components of processor 700, for example the controller 714, cache 710, and/or register 712. The bus 716 may be coupled to one or more components of the system.

The bus 716 may be coupled to one or more external memories. The external memories may include Read Only Memory (ROM) 732. ROM 732 may be a masked ROM, Electronically Programmable Read Only Memory (EPROM) or any other suitable technology. The external memory may include Random Access Memory (RAM) 733. RAM 733 may be a static RAM, battery backed up static RAM, Dynamic RAM (DRAM) or any other suitable technology. The external memory may include Electrically Erasable Programmable Read Only Memory (EEPROM) 735. The external memory may include Flash memory 734. The external memory may include a magnetic storage device such as disc 736. In some embodiments, the external memories may be included in a system.

The invention can be implemented in any suitable form including hardware, software, firmware or any combination of these. The invention may optionally be implemented at least partly as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit or may be physically and functionally distributed between different units, circuits and processors.

Although the present invention has been described in connection with some embodiments, it is not intended to be limited to the specific form set forth herein. Rather, the scope of the present invention is limited only by the accompanying claims. Additionally, although a feature may appear to be described in connection with particular embodiments, one skilled in the art would recognize that various features of the described embodiments may be combined in accordance with the invention. In the claims, the term comprising does not exclude the presence of other elements or steps.

Furthermore, although individually listed, a plurality of means, elements, circuits or method steps may be implemented by e.g., a single circuit, unit or processor. Additionally, although individual features may be included in different claims, these may possibly be advantageously combined, and the inclusion in different claims does not imply that a combination of features is not feasible and/or advantageous. Also, the inclusion of a feature in one category of claims does not imply a limitation to this category but rather indicates that the feature is equally applicable to other claim categories as appropriate. Furthermore, the order of features in the claims does not imply any specific order in which the features must be worked, and in particular, the order of individual steps in a method claim does not imply that the steps must be performed in this order. Rather, the steps may be performed in any suitable order. In addition, singular references do not exclude a plurality. Thus, references to "a", "an", "first", "second" etc. do not preclude a plurality. Reference signs in the claims are provided merely as a clarifying example shall not be construed as limiting the scope of the claims in any way.

## Claims

1. An apparatus for generating a biomedical data signal, the apparatus comprising:
a receiver (101) arranged to receive a plurality of biomedical signals;
a cluster circuit (109) arranged to generate a set of clusters of biomedical signals by applying a clustering process to the plurality of biomedical signals, the clustering process being arranged to cluster biomedical signals of the plurality of biomedical signals depending on signal property similarities between the plurality of biomedical signals;
a data encoder (103) arranged to generate encoded compressed data by encoding the plurality of biomedical signals, the data encoder (103) being arranged to apply relative encoding to biomedical signals within a first cluster of the set of clusters; and
a data signal generator (105) arranged to generate the biomedical data signal to comprise the compressed encoded data and an indication of the set of clusters.

2. The apparatus of claim 1 wherein the cluster circuit (109) is arranged to repeatedly perform the clustering process to update the set of clusters.

3. The apparatus of claim 1 comprising a transform circuit arranged to apply a signal transform to the plurality of biomedical signals prior to the clustering process.

4. The apparatus of any previous claim arranged to apply a sequence of operations to the plurality of biomedical signals to generate the encoded compressed data; and wherein the data signal generator (105) is arranged to include an indication of a position of the clustering process in the sequence.

5. The apparatus of any previous claim wherein the plurality of biomedical signals comprise biomedical signals of at least two different types.

6. The apparatus of any previous claim wherein the clustering process is arranged to cluster the biomedical signals of the plurality of biomedical signals depending on measurement positions for the plurality of biomedical signals.

7. The apparatus of any previous claim wherein the data encoder (103) is arranged to individually for at least two clusters of the set of clusters select an encoding process from a plurality of encoding processes in dependence on signal properties of biomedical signals comprised in the at least two clusters.

8. The apparatus of any previous claim wherein the data encoder is arranged to time align biomedical signals of at least a first cluster of the set of clusters.

9. The apparatus of any previous claim wherein the cluster circuit (109) is arranged to select the clustering process from a set of candidate clustering processes, and the data signal generator (105) is arranged to generate the biomedical data signal to comprise an indication of the clustering process.

10. The apparatus of any previous claim further comprising:
a determination circuit (601) arranged to derive a first reference signal from biomedical signals of a first cluster of the set of clusters;
a compensation circuit (603) arranged to generate compensated biomedical signals by compensating at least some of the biomedical signals of a first cluster for the first reference signal; and wherein the data encoder (103) is arranged to generate encoded compressed data for the first cluster by compressing the at least some of the biomedical signals of the first cluster.

11. An apparatus for generating a plurality of biomedical signals, the apparatus comprising:
a receiver (201) arranged to receive a biomedical data signal comprising compressed encoded data for a plurality of biomedical signals and a cluster indication indicative of a clustering of the plurality of biomedical signals into a set of clusters, each cluster comprising biomedical signals clustered in dependence on signal property similarities between the plurality of biomedical signals, the encoded data comprising relative encoding data for at least some biomedical signals within a same cluster; and
a decoder arranged to generate the plurality of biomedical signals by decoding the encoded data in dependence on the cluster indication.

12. A method of generating a biomedical data signal, the method comprising:
receiving a plurality of biomedical signals;
generating a set of clusters of biomedical signals by applying a clustering process to the plurality of biomedical signals, the clustering process being arranged to cluster biomedical signals of the plurality of biomedical signals depending on signal property similarities between the plurality of biomedical signals;
generating encoded compressed data by encoding the plurality of biomedical signals, the data encoder being arranged to apply relative encoding to biomedical signals within a first cluster of the set of clusters; and
generating the biomedical data signal to comprise the compressed encoded data and an indication of the set of clusters.

13. A method of generating a plurality of biomedical signals, the method comprising:
receiving a biomedical data signal comprising compressed encoded data for a plurality of biomedical signals and a cluster indication indicative of a clustering of the plurality of biomedical signals into a set of clusters, each cluster comprising biomedical signals clustered in dependence on signal property similarities between the plurality of biomedical signals, the encoded data comprising relative encoding data for at least some biomedical signals within a same cluster; and
generating the plurality of biomedical signals by decoding the encoded data in dependence on the cluster indication.

14. A biomedical data signal for representing a plurality of biomedical signals, the biomedical data signal comprising:
compressed encoded data for a plurality of biomedical signals; and
a cluster indication indicative of a clustering of the plurality of biomedical signals into a set of clusters, each cluster comprising biomedical signals clustered in dependence on signal property similarities between the plurality of biomedical signals; and wherein
the encoded data comprise relative encoding data for at least some biomedical signals within a same cluster.

15. A computer program product comprising computer program code means adapted to perform all the steps of claims 12 or 13 when said program is run on a computer, or comprising the biomedical data signal of claim **14.**
